(19) Europäisches Patentamt
European Patent Office
Office européen des brevets

(11) **EP 1 726 593 B1**

(12) **EUROPÄISCHE PATENTSCHRIFT**

(45) Veröffentlichungstag und Bekanntmachung des Hinweises auf die Patenterteilung:
**25.07.2007   Patentblatt 2007/30**

(51) Int Cl.:
***C07F 5/02*** *(2006.01)*

(21) Anmeldenummer: 06016494.4

(22) Anmeldetag: **15.01.2004**

(54) **Salze mit Cyanoborat-Anionen**

Salts comprising cyanoborate anions

Sels base d'anions cyanoborates

(84) Benannte Vertragsstaaten:
**AT BE BG CH CY CZ DE DK EE ES FI FR GB GR HU IE IT LI LU MC NL PT RO SE SI SK TR**

(30) Priorität: **14.02.2003   DE 10306617**

(43) Veröffentlichungstag der Anmeldung:
**29.11.2006   Patentblatt 2006/48**

(62) Dokumentnummer(n) der früheren Anmeldung(en) nach Art. 76 EPÜ:
**04702317.1 / 1 592 696**

(73) Patentinhaber: **Merck Patent GmbH**
**64293 Darmstadt (DE)**

(72) Erfinder:
• **Welz-Biermann, Urs Dr.**
**64646 Heppenheim (DE)**
• **Ignatiev, Nikolai Dr.**
**47058 Duisburg (DE)**
• **Bernhardt, Eduard Dr.**
**42107 Wuppertal (DE)**
• **Finze, Maik Dr.**
**31582 Nienburg (DE)**
• **Willner, Helge Professor Dr.**
**45481 Muelheim/Ruhr (DE)**

(56) Entgegenhaltungen:
• **WILLIAMS, DARRICK ET AL: "Synthesis of LiBC4N4, BC3N3, and Related C-N Compounds of Boron: New Precursors to Light Element Ceramics" JOURNAL OF THE AMERICAN CHEMICAL SOCIETY , 122(32), 7735-7741 CODEN: JACSAT; ISSN: 0002-7863, 2000, XP002278152**
• **BERNHARDT, E. ET AL: 'The tetracyanoborates M[B(CN)4], M = [Bu4N]+, Ag+, K+' ZEITSCHRIFT FUER ANORGANISCHE UND ALLGEMEINE CHEMIE Bd. 626, Nr. 2, 2000, Seiten 560 - 568, XP009030010**
• **BERNHARDT, E. ET AL: 'An efficient synthesis for tetracyanoborates by sinter processes' ZEITSCHRIFT FUER ANORGANISCHE UND ALLGEMEINE CHEMIE Bd. 629, Nr. 7-8, 2003, Seiten 1229 - 1234, XP009030008**

EP 1 726 593 B1

**Beschreibung**

Die vorliegende Erfindung betrifft ein Verfahren zur Herstellung von Alkalimetallcyanoboraten.

Ionische Flüssigkeiten oder flüssige Salze sind ionische Spezies, die aus einem organischen Kation und einem i.d.R. anorganischen Anion bestehen. Sie enthalten keine neutralen Moleküle, und weisen in der Regel Schmelzpunkte kleiner 373 K auf. Im Stand der Technik sind eine Vielzahl von Verbindungen bekannt, die als ionische Flüssigkeiten Verwendung finden. Insbesondere sind sie auch Gegenstand einer Reihe von Patenten bzw. Patentanmeldungen.

So wurden lösungsmittelfreie ionische Flüssigkeiten erstmals von Hurley und Wier in einer Reihe von US-Patenten (US 2,446,331, US 2,446,339 und US 2,446,350) offenbart. Diese "bei Raumtemperatur geschmolzenen Salze" enthielten $AlCl_3$ und eine Vielzahl von n-Alkylpyridinium-Halogeniden.

In den letzten Jahren wurden einige Übersichtsartikel zu diesem Thema veröffentlicht (R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083; R. Hagiwara, Ya. Ito "Room temperature ionic liquids of alkylimidazolium cations and fluoroanions", Journal of Fluorine Chem., 105 (2000), 221-227).

Die Eigenschaften ionischer Flüssigkeiten, z.B. Schmelzpunkt, thermische und elektrochemische Stabilität, Viskosität, werden stark von der Natur des Anions beeinflusst. Demgegenüber können die Polarität und die Hydrophilie bzw. Lipophilie durch die geeignete Wahl des Kation/Anion-Paares variiert werden. Daher besteht grundsätzlicher Bedarf an neuen ionischen Flüssigkeiten mit variierten Eigenschaften, die zusätzliche Möglichkeiten hinsichtlich ihrer Verwendung ermöglichen.

Entscheidende Fortschritte auf dem Gebiet der ionischen Flüssigkeiten wurden mit der Entdeckung des 1-Ethyl-3-methylimidazolium Chloroaluminats erreicht. Dieses Salz hat einen breiten Flüssigbereich und ein elektrochemisches Fenster von mehr als 3 V und ist somit von großem Interesse für elektrochemische und synthetische Zwecke. Seine Anwendung ist aber durch die chemische Instabilität, vor allem gegen Feuchtigkeit, begrenzt. Nach der Entdeckung des hydrolysestabileren 1-Ethyl-3-methylimidazolium Tetrafluorborats wurden Kombinationen von Alkylimidazolium-Kationen mit anorganischen oder organischen Anionen untersucht, von denen das 1-Ethyl-3-methylimidazolium Tetrafluorborat am besten charakterisiert ist.

Die Stabilität des Imidazolium-Kations ist relativ hoch und seine Zersetzungstemperatur wird im wesentlichen durch das Anion bestimmt. So sind die 1-Ethyl-3-methylimidazolium-Salze mit Triflat- und Bis(trifluormethylsulfonyl)imid-Anionen bis 400°C stabil, wohingegen das 1-Ethyl-3-methylimidazolium Tetrafluorborat nur bis 300°C stabil ist.

Im Stand der Technik sind Borat-Anionen beschrieben, bei denen Fluor-Liganden durch Cyanid (E. Bernhardt, G. Henkel, H. Willner, Z. Anorg. Allg. Chem. 626 (2000) 560; D. Williams, B. Pleune, J. Kouvetakis, M. D. Williams, R. A. Andersen, J. Amer. Chem. Soc. 122 (2000) 7735; E. Bernhardt, M. Berkei, M. Schürmann, H. Willner, Z. Anorg. Allg. Chem. 628 (2002) 1734) und Trifluormethyl-Liganden (E. Bernhardt, G. Henkel, H. Willner, G. Pawelke, H. Bürger, Chem. Eur. J. 7 (2001) 4696; G. Pawelke, H. Bürger, Coord. Chem. Rev. 215 (2001) 243) ausgetauscht sind. Dabei werden die Trifluormethyl-Borate ausgehend von den Cyanoboraten synthetisiert, wobei allerdings die Cyanoborate schwer und nur in geringen Mengen zugänglich sind. Die Synthese von $[B(CN_4)]^-$ ist arbeitsintensiv und nur in kleinem präparativem Maßstab durchführbar. Darüber hinaus sind die Ausgangsmaterialien teuer.

Aufgabe der vorliegenden Erfindung ist es, ein effektives und wirtschaftlich sinnvolles Verfahren zur Herstellung dieser Borat-Salze und ihrer Vorläufer zur Verfügung zu stellen.

Diese Aufgabe wird erfindungsgemäß durch die kennzeichnenden Merkmale des Hauptanspruchs und der nebengeordneten Ansprüche gelöst.

Der Gegenstand der vorliegenden Erfindung ist ein Verfahren zur Herstellung von Alkalimetallcyanoboraten der allgemeinen Formel (2)

$$M^+ [BF_n(CN)_{4-n}]^- \qquad (2),$$

wobei n = 0, 1, 2 oder 3 und

M ausgewählt ist aus der Gruppe Li, Na, K, Rb und Cs,

bei dem ein Alkalimetallcyanid MCN, wobei M = Li, Na, K, Rb, Cs, mit Bortrifluorid-Etherat $BF_3 \cdot OEt_2$ umgesetzt wird.

Bei der Verwendung von grobkörnigem Kaliumcyanid KCN und $BF_3 \cdot OEt_2$ bilden sich bei der erfindungsgemäßen Umsetzung neben dem primären Adukt $K[BF_3(CN)]$ auch äquimolare Mengen von $K[BF_4]$ und $K[BF_2(CN)_2]$ gemäß der folgenden Gleichungen:

$$K\big[\overset{-}{BF_3(CN)}\big] \ + \ BF_3 \cdot OEt_2 \ \rightleftharpoons \ K\big[BF_4\big] \ + \ BF_2(CN) \cdot OEt_2$$

$$BF_2(CN) \cdot OEt_2 + KCN \rightarrow K[BF_2(CN)_2] + Et_2O$$

Daneben bilden sich in geringem Umfang die beiden Salze K[BF(CN)$_3$] und K[B(CN)$_4$], ersteres insbesondere wenn die Reaktionsmischung bei Temperaturen über Raumtemperatur getempert wird.

Erfindungsgemäß wird das Bortrifluorid-Etherat mit dem Alkalimetallcyanid in Gegenwart eines aprotischen Lösungsmittels umgesetzt. Ohne Einschränkung der Allgemeinheit kann das aprotische Lösungsmittel beispielsweise Acetonitril, Diethylether, Tetrahydrofuran und/oder Dimethoxyethan sein.

Für das erfindungsgemäße Verfahren wird als Alkalimetallcyanid vorzugsweise Kaliumcyanid KCN verwendet.

Die Umsetzung der Edukte erfolgt erfindungsgemäß bevorzugt bei Temperaturen von -80 bis 100°C, besonders bevorzugt bei Raumtemperatur.

Bei der Reaktion können flüchtige Nebenprodukte entstehen, die unter Vakuum entfernt werden. Meistens bilden sich jedoch in den verwendeten Lösungsmitteln unlösliche Nebenprodukte, die durch Filtration abgetrennt werden. Das Lösungsmittel wird gegebenenfalls zusammen mit flüchtigen Nebenprodukten unter reduziertem Druck entfernt und die erhaltenen Alkalimetallcyanoborate können nach einer dem Fachmann bekannten gängigen Möglichkeit gegebenenfalls getrennt und gereinigt werden.

Die NMR-Spektren wurden an Lösungen in deuterierten Lösungsmitteln bei 20°C an einem Bruker Avance DRX-300 Spektrometer mit einem 5 mm Breitbandkopf $^1$H/BB mit Deuterium Lock gemessen. Die Messfrequenzen der verschiedenen Kerne sind: $^1$H: 300, 13 MHz, $^{11}$B: 96,92 MHz, $^{13}$C: 75,47 MHz, $^{19}$F: 282,41 MHz und $^{15}$N: 30,41 MHz. Die Methode der Referenzierung wird bei jedem Spektrum bzw. bei jedem Datensatz separat angegeben.

DSC-Messungen wurden an einem Netzsch DSC 204 Gerät durchgeführt. Zur Kalibrierung der Temperatur und der Empfindlichkeit fanden Naphthalin, Benzoesäure, KNO$_3$, AgNO$_3$, LiNO$_3$ und CsCl Verwendung. Von den Substanzen wurden jeweils 5-20 mg in einen Aluminium-Tiegel eingewogen und mit Aluminium-Kappen mit einer kleinen Öffnung verschlossen. Die Untersuchung erfolgte im Temperaturbereich von 25 bis 500°C. Sofern nicht anders angegeben beträgt die Aufheizrate 10 Kmin$^{-1}$. Während der Messung wurde der Probenraum mit trockenem Stickstoff gespült. Die Proben luftempfindlicher Substanzen wurden in einer Trockenbox präpariert und in einem mit Argon gefüllten Gläschen zum Analysengerät transportiert. Die Datenauswertung wurde mit dem Programm Netzsch Protens 4.0 vorgenommen.

Die Elementaranalysen erfolgten nach den Verbrennungsmethoden der Mikroanalytik mit einem Euro EA3000 der Firma HEKA-Tech GmbH. Die Proben luftempfindlicher Substanzen wurden in einer Trockenbox präpariert und in einem mit Argon gefüllten Gläschen zum Analysengerät transportiert. Die Fehlergrenzen für die registrierten Atome betragen: C: $\pm$0,3 %, H: $\pm$0,1 %, N: $\pm$0,2 %.

Beispiel 11: Darstellung von K[BF$_2$(CN)$_2$]

*Variante A:* Zu 4.12 g (63 mmol) KCN werden in einem 50 mL Kolben mit PTFE-Ventil 5.88 g (41 mmol) BF$_3$·OEt$_2$ und 30 mL CH$_3$CN aufkondensiert. Das Reaktionsgemisch wird 3 h bei Raumtemperatur gerührt und anschließend werden alle flüchtigen Bestandteile im Vakuum entfernt, der Rückstand im ca. 50 mL CH$_3$CN gelöst und von KCN und K[BF$_4$] durch Filtration befreit. Nach dem Entfernen des Acetonitrils im Vakuum werden 2.66 g (19 mmol) K[BF$_2$(CN)$_2$] ([11]Bund $^{19}$F-NMR: 93% [BF$_2$(CNh)$_2$]$^-$, 0.3% [BF$_3$(CN)]$^-$ und ca. 7% unbekannte Spezies) erhalten. Ausbeute: 92%. Durch Umkristallisieren aus Wasser wird reines farbloses K[BF$_2$(CN)$_2$] erhalten. Isolierte Ausbeute: 2.08 g (72%, 15 mmol).

*Variante B:* In einem 500 mL Rundkolben mit Tropftrichter werden 65 g (1.0 mol) KCN und 200 mL CH$_3$CN vorgelegt. Bei Raumtemperatur werden innerhalb von einer halben Stunde unter Rühren 50 mL (56 g, 0.4 mol) BF$_3$·OEt$_2$ zugetropft. Während der Zugabe steigt die Temperatur auf 50 °C. Nach weiterem Rühren (1.5 h) bei Raumtemperatur wird die Lösung abfiltriert und der Filterrückstand (KCN und K[BF$_4$]) mit ca. 300 mL CH$_3$CN gewaschen. Die vereinigten Acetonitril Phasen werden am Rotationsverdampfer eingeengt. Es werden als Rohprodukt 20 g verunreinigtes K[BF$_2$(CN)$_2$] erhalten. In 200 mL Wasser wird das Rohprodukt mit 30 mL konz. HCl und 35mL (25 g, 170 mmol) Tripropylamin umgesetzt und mit 200 mL Dichlormethan als Tripropylammoniumsalz extrahiert. Die Dichlormethanphase wird mit MgSO$_4$ getrocknet und mit 25 g KOH gelöst in möglichst wenig Wasser unter starkem Rühren umgesetzt. Die zähflüssige wässrige Phase wird abgetrennt und mit Dichlormethan gewaschen. Aus dem Rückstand wird mit ca. 300 mL CH$_3$CN das Produkt extrahiert, die Lösung mit K$_2$CO$_3$ getrocknet und am Rotationsverdampfer eingeengt. Das weiße Produkt wird mit Dichlormethan gewaschen und im Vakuum getrocknet. Ausbeute: 17g (60%, 120 mmol). Laut $^{11}$B-NMR enthält die Substanz 98 % [BF$_2$(CN)$_2$]$^-$.

**Patentansprüche**

1. Verfahren zur Herstellung von Alkalimetallcyanoboraten der allgemeinen Formel (2)

$$M^+ [BF_n(CN)_{4-n}]^- \qquad (2),$$

wobei n = 0, 1, 2 oder 3
und M ausgewählt ist aus der Gruppe Li, Na, K, Rb und Cs,
**dadurch gekennzeichnet, dass** ein Alkalimetallcyanid MCN mit Bortrifluorid-Etherat $BF_3 \cdot OEt_2$ umgesetzt wird.

2.  Verfahren nach Anspruch 1,
    **dadurch gekennzeichnet, dass** das Alkalimetallcyanid mit dem Bortrifluorid-Etherat in Gegenwart eines aprotischen Lösungsmittels umgesetzt wird.

3.  Verfahren nach Anspruch 1 oder 2,
    **dadurch gekennzeichnet, dass** das Alkalimetallcyanid mit dem Bortrifluorid-Etherat in Gegenwart von Acetonitril, Diethylether, Tetrahydrofuran und/oder Dimethoxyethan umgesetzt wird.

4.  Verfahren nach einem oder mehreren der Ansprüche 1 bis 3,
    **dadurch gekennzeichnet, dass** als Alkalimetallcyanid Kaliumcyanid KCN eingesetzt wird.

5.  Verfahren nach einem oder mehreren der Ansprüche 1 bis 4,
    **dadurch gekennzeichnet, dass** die Umsetzung bei Temperaturen von -80 bis 100°C, bevorzugt bei Raumtemperatur, durchgeführt wird.

**Claims**

1.  Process for the preparation of alkali metal cyanoborates of the general formula (2)

$$M^+ [BF_n(CN)_{4-n}]^- \qquad (2),$$

where n = 0, 1, 2 or 3
and M is selected from the group Li, Na, K, Rb and Cs,
**characterised in that** an alkali metal cyanide MCN is reacted with boron trifluoride etherate $BF_3OEt_2$.

2.  Process according to Claim 1,
    **characterised in that** the alkali metal cyanide is reacted with the boron trifluoride etherate in the presence of an aprotic solvent.

3.  Process according to Claim 1 or 2,
    **characterised in that** the alkali metal cyanide is reacted with the boron trifluoride etherate in the presence of acetonitrile, diethyl ether, tetrahydrofuran and/or dimethoxyethane.

4.  Process according to one or more of Claims 1 to 3,
    **characterised in that** the alkali metal cyanide employed is potassium cyanide KCN.

5.  Process according to one or more of Claims 1 to 4,
    **characterised in that** the reaction is carried out at temperatures of -80 to 100°C, preferably at room temperature.

**Revendications**

1.  Procédé de préparation de cyanoborates de métal alcalin de formule générale (2)

$$M^+ [BF_n(CN)_{4-n}]^- \qquad (2),$$

dans laquelle n = 0, 1, 2 ou 3
et M est choisi parmi le groupe constitué par Li, Na, K, Rb et Cs,
**caractérisé en ce qu'**un cyanure de métal alcalin MCN est réagi avec de l'éthérate de trifluorure de bore $BF_3 \cdot OEt_2$.

**2.** Procédé selon la revendication 1,
**caractérisé en ce que** le cyanure de métal alcalin est réagi avec l'éthérate de trifluorure de bore en présence d'un solvant aprotique.

**3.** Procédé selon la revendication 1 ou 2,
**caractérisé en ce que** le cyanure de métal alcalin est réagi avec l'éthérate de trifluorure de bore en présence d'acétonitrile, d'éther diéthylique, de tétrahydrofurane et/ou de diméthoxyéthane.

**4.** Procédé selon l'une ou plusieurs parmi les revendications 1 à 3,
**caractérisé en ce que** le cyanure de métal alcalin employé est le cyanure de potassium KCN.

**5.** Procédé selon l'une ou plusieurs parmi les revendications 1 à 4,
**caractérisé en ce que** la réaction est réalisée à des températures allant de -80 à 100°C, préférablement à température ambiante.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2446331 A
- US 2446339 A
- US 2446350 A

**In der Beschreibung aufgeführte Nicht-Patentliteratur**

- **R. SHELDON.** Catalytic reactions in ionic liquids. *Chem. Commun.,* 2001, 2399-2407
- **M.J. EARLE ; K.R. SEDDON.** Ionic liquids. Green solvent for the future. *Pure Appl. Chem.,* 2000, vol. 72, 1391-1398
- **P. WASSERSCHEID ; W. KEIM.** Ionische Flüssig-keiten - neue Lösungen für die Übergangsmetallk-atalyse. *Angew. Chem.,* 2000, vol. 112, 3926-3945
- **T. WELTON.** Room temperature ionic liquids. Solvents for synthesis and catalysis. *Chem. Rev.,* 1999, vol. 92, 2071-2083
- **R. HAGIWARA ; YA. ITO.** Room temperature ionic liquids of alkylimidazolium cations and fluoroanions. *Journal of Fluorine Chem.,* 2000, vol. 105, 221-227
- **E. BERNHARDT ; G. HENKEL ; H. WILLNER.** *Z. Anorg. Allg. Chem.,* 2000, vol. 626, 560
- **D. WILLIAMS ; B. PLEUNE ; J. KOUVETAKIS ; M. D. WILLIAMS ; R. A. ANDERSEN.** *J. Amer. Chem. Soc.,* 2000, vol. 122, 7735
- **E. BERNHARDT ; M. BERKEI ; M. SCHÜRMANN ; H. WILLNER.** *Z. Anorg. Allg. Chem.,* 2002, vol. 628, 1734
- **E. BERNHARDT ; G. HENKEL ; H. WILLNER ; G. PAWELKE ; H. BÜRGER.** *Chem. Eur. J.,* 2001, vol. 7, 4696
- **G. PAWELKE ; H. BÜRGER.** *Coord. Chem. Rev.,* 2001, vol. 215, 243